# EUROPEAN PATENT SPECIFICATION

(11) **EP 3 325 463 B1**
(45) Date of publication and mention of the grant of the patent: **05.06.2019**
(21) Application number: 16744468.6
(22) Date of filing: 19.07.2016
(51) Int. Cl.: C07D 339/08, C07D 409/04, H01L 51/00

(54) **HETEROCYCLIC COMPOUNDS AND THEIR USE IN ELECTRO-OPTICAL OR OPTO-ELECTRONIC DEVICES**
HETEROCYCLISCHE VERBINDUNGEN UND DEREN VERWENDUNG IN ELEKTROOPTISCHEN ODER OPTOELEKTRONISCHEN VORRICHTUNGEN
COMPOSÉS HÉTÉROCYCLIQUES ET LEUR UTILISATION DANS DES DISPOSITIFS ÉLECTRO-OPTIQUES OU OPTO-ÉLECTRONIQUES

(30) Priority: 20.07.2015 GB 201512720
(43) Date of publication of application: 30.05.2018
(73) Proprietor: Power Oleds Limited, London NW2 1EL (GB)
(72) Inventor: KATHIRGAMANATHAN, Poopathy, North Harrow Middlesex HA2 7NN (GB)
(74) Representative: Lucas, Phillip Brian
(86) International application number: PCT/GB2016/052176
(87) International publication number: WO 2017/013420

(56) References cited:
- WO-A1-2010/002848
- WO-A2-2014/167286
- US-A1- 2013 175 507
- US-A1- 2013 241 401
- US-A1- 2014 077 175

## Description

### FIELD OF THE INVENTION

This invention relates to novel compounds and to their use in electro-optical or opto-electronic devices, *inters alia* optical light emitting devices, for example in a hole transport layer.

### BACKGROUND TO THE INVENTON

### Hole transport materials

One class of hole transport materials comprises aromatic tertiary amines including at least two aromatic tertiary amine moieties (e.g. those based on biphenyl diamine or of a "starburst" configuration), of which the following are representative and of which at this time α-NPB [Tg (°C) 98, µh (cm² V^{-1 s-1}) 1 x 10⁻⁴] is believed to be the most widely accepted and used in commercial production.

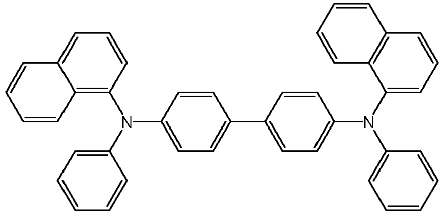

WO 2014/167286 (Kathirgamanathan, the disclosure of which is incorporated herein by reference) is concerned with the problem of providing further, or in the alternative improved, compounds with hole transport properties and discloses *inter alia* hole transport materials having one, two or three thianthrene moieties linked to conjugated or aromatic hydrocarbon other than alkyl-substituted fluorine. Embodiments of the present compounds are said to exhibit a surprisingly favourable combination of hole mobility and high glass transition temperature or melting point, so that they may find utility in a hole injection layer and/or in an electron blocking layer. Since they are in general small molecules, many of them are purifiable by sublimation, which is desirable for the production of compounds of the purity required for OLEDs and other device applications. Specific compounds reported include: 4-(Thianthren-1-yl)triphenylamine (also diphenyl-(4-thianthren-1-yl-phenyl)amine) (HTS-1); 4,4',4"-tri-(thianthren-1-yl)triphenylamine (also tris-(4-thianthren-1-yl-phenyl)-amine; HTS-2); 9-(4-Thianthren-1-yl-phenyl)-9H-carbazole (HTS-3); 4,4'-di-(thianthren-2-yl)triphenylamine (phenyl-bis(4-thianthren-1-yl-phenyl)-amine; HTS-4); 4-(10H-phenothiazin-10-yl)triphenylamine (N-(4-(10H-phenothiazin-10-yl)phenyl)-N-phenylbenzenamine; HTS-5); and 2,8-Bis(1-thianthrenyl)dibenzothiophene [HTS-8]. HOMO and LUMO levels compared to α-NPB are shown below.

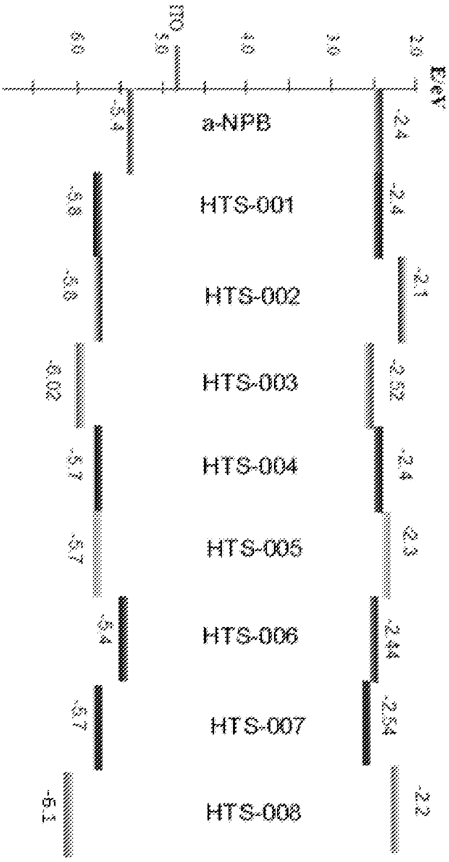

The general teachings in Kathirgamanathan of cell structure, anodes, hole injectors and hole transport layers, hole injection materials, electroluminescent materials, electron transport materials, electron injection materials and cathodes are incorporated herein by reference in their entirety.

### SUMMARY OF THE INVENTION

The present problem provides additional, and it is believed improved, thianthrene-based compounds of the kind described in WO 2014/167286.

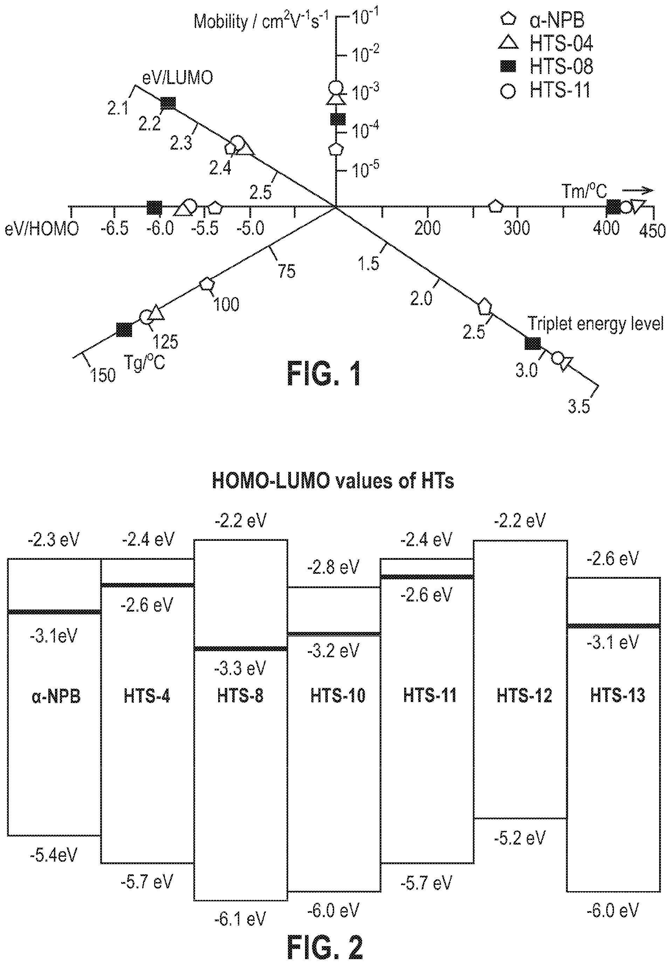

In one aspect the invention provides the compound 2-(4'-diphenylamino)phenyl-8-(1'-thianthrenyl)-dibenzothiophene. Also disclosed is compound 4-(1-thianthrenyl)-bis(triphenylamine). As apparent in Fig. 1, these compounds have favourable physical properties including HOMO levels, LUMO levels and triplet levels.

In a further aspect the invention provides a hole transport material comprising 2-(4'-diphenylamino)phenyl-8-(1'-thianthrenyl)-dibenzothiophene and a p-dopant which is an acceptor-type organic molecule. In some embodiments the dopant is present in an amount such that when the material is deposited to form a layer the dopant contributes about 10-40% to the layer thickness, e.g. about 33%. Other materials that may be present in the composition, in embodiments in minor amounts e.g. <50 wt% based on the weight of the above compound or mixture, include known organic hole transport agents e.g. an aromatic tertiary amine of biphenyl or starburst configuration, in some embodiments α-NPB.

In a further aspect the invention provides an optical light-emitting diode having first and second electrodes and between said electrodes a layer comprising 2-(4'-diphenylamino)phenyl-8-(1'-thianthrenyl)-dibenzothiophene or a material as defined above. The layer may be a hole transport layer or a hole injection layer. The device may form part of a flat panel display or a lighting panel. Other end-uses may include organic photovoltaic devices, imaging members for forming an electrostatic latent image, organic thin film transistors and dye-sensitised solar cells.

The above compounds may be incorporated into other opto-electronic or electro-optic devices as described below.

### BRIEF DESCRIPTION OF THE DRAWINGS

How the invention mat be put into effect will now be described with reference to the accompanying drawings, in which:
Fig. 1 is a diagram showing plots along variously directed lines of hole mobility, melting point, glass transition temperature, HOMO level and LUMO level for the compounds α-NPB, HTS-4, HTS-8 and HTS-11;
Fig 2 is a bar chart showing HOMO and LUMO levels for various hole transport compounds;
Fig 3 shows luminous efficiency for various OLED devices of Example 4; and
Fig. 4 is an energy band diagram for the OLED device of Example 4

### DETAILED DESCRIPTION OF PREFERRED EMBODIMENTS

The above compounds may be purified by sublimation. Their physical properties and those of related materials are summarised in the table below:

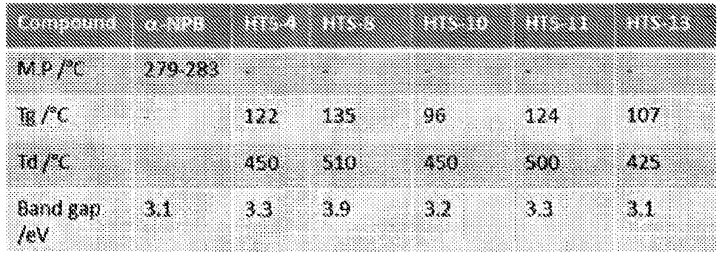

The above compounds may be used as hole transporters on their own or may comprise a hole transport material together with p-dopant which is an acceptor-type organic molecule. In the latter case, the dopant may be present in an amount such that when the material is deposited to form a layer the dopant contributes about 10-40% to layer thickness, e.g. in an amount such that when the material is deposited to form a layer the dopant contributes about 33% to layer thickness. The dopant may comprise tetracyanoquinodimethane or tetrafluorotetracyano-quinodimethane or it may comprise a compound of any
of the general formulae (i)-(v) below: 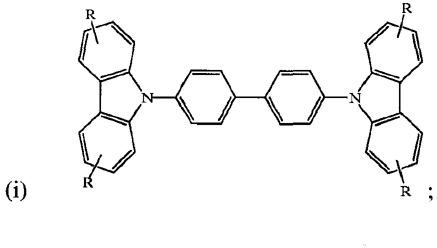 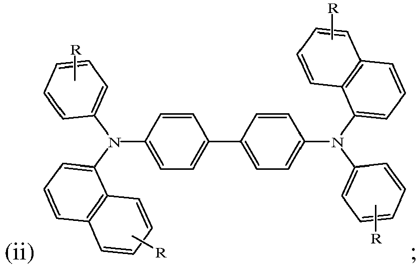 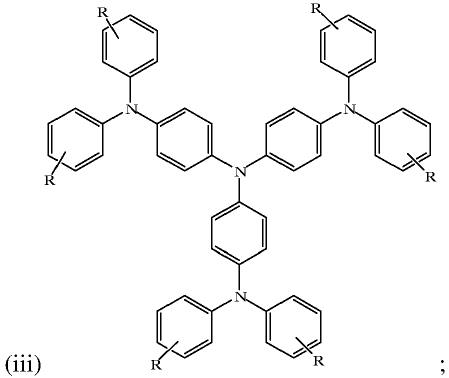 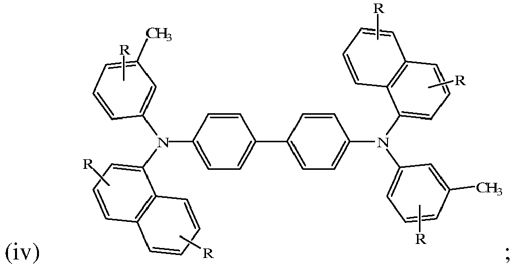 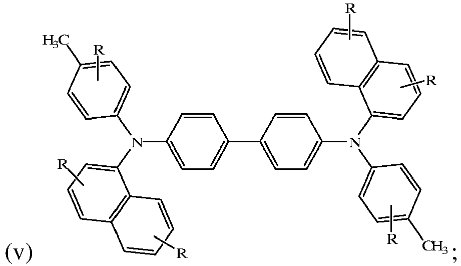 wherein the groups R in any of the formulae in (i) to (v) can be the same or different and are selected from hydrogen; substituted and unsubstituted aliphatic groups; substituted and unsubstituted aromatic, heterocyclic and polycyclic ring structures; halogens; and thiophenyl groups; and wherein
in formula (i) the methyl groups may be replaced by C₁-C₄ alkyl or monocyclic or polyclic aryl or heteroraryl which may be further substituted e.g. with alkyl, aryl or arylamino, or of the formula viii or ix 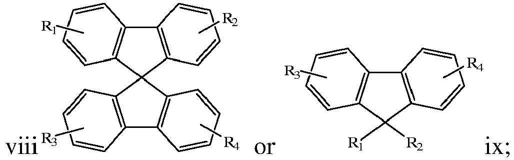 wherein the groups R₁-R₄ when appearing in either of the above formulae can be the same or different and are selected from hydrogen; substituted and unsubstituted aliphatic groups; substituted and unsubstituted aromatic, heterocyclic and polycyclic ring structures; halogens; and thiophenyl groups.

Such dopants may comprise a teritiary amine which is α-NPB or β-NPB.

The above compounds may be incorporated into an optical light-emitting diode OLED) having first and second electrodes and between said electrodes a layer comprising a compound as set out above or a doped material material as set out above, either compound or a mixture of them forming a hole transport layer. In such anOLED the emissive layer may be a fluorescent emitter, a phosphorescent emitter, an ion fluorescent (rare earth based) emitter, a quantum dot or a thermally activated fluorescent (TADF) material.

In such a device there may be provided a hole injection layer comprising PEDOT:PSS, CuPC, ZnTpTP, 2-TNATA, MoO₃ or

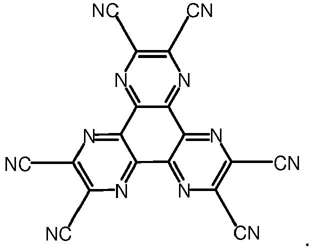

Known electron transport materials may provide an electron transport layer e.g. aluminium quinolate, zirconium quinolate. Organic electron transporters which may be used include 1,3,5-tris(N-phenylbenzimidizol-2-yl)benzene and any of the compounds shown below:

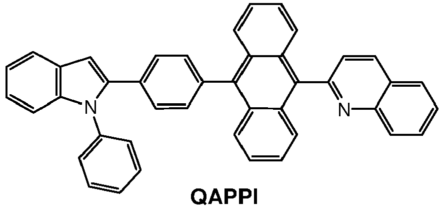

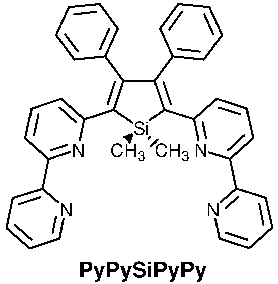

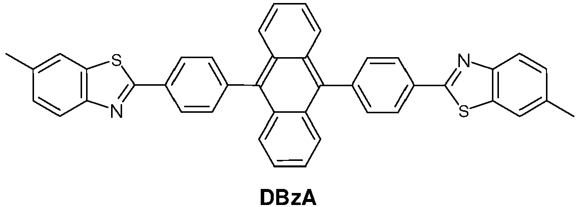

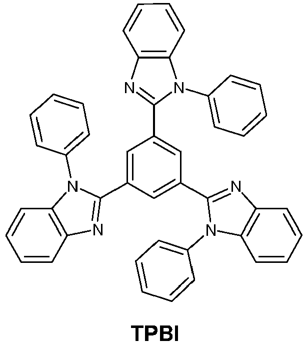

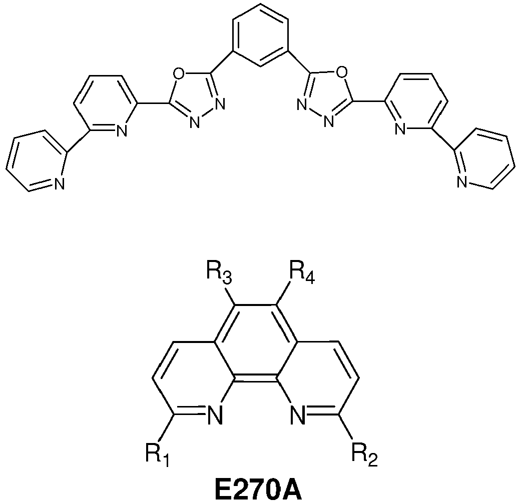

As electron injection layer, any known electron injection material may be used, LiF being typical. Other possibilities include BaF2, CaF2, CsF,MgF2 and KF. The electron injection layer may be deposited direct onto the cathode and may also comprise a compound of the formula 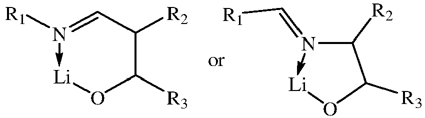 wherein
R₁ is a 1-5 ring aryl (including polycyclic aryl or aryl-substituted polycyclic aryl), aralkyl or heteroaryl group which may be substituted with one or more C₁-C₄ alkyl or alkoxy substituents; and
R₂ and R₃ together form a 1-5 ring aryl (including polycyclic or aryl-substituted polycyclic aryl), aralkyl or heteroaryl group which may be substituted with one or more C₁-C₄ alkyl or alkoxy substituents. A compound of the above formula may be used alone or in combination with another electron injection material e.g. a quinolate such as lithium or zirconium quinolate. The Schiff base preferably comprises at least 30 wt% of the electron injection layer, more preferably at least 50 wt%.

In the formula set out above, R₁ may be polycyclic aryl e.g. naphthyl, anthracenyl, tetracenyl, pentacenyl or a perylene or pyrene compound or may have up to 5 aromatic rings arranged in a chain e.g. biphenyl. It is preferably phenyl or substituted phenyl. R₂ and R₃ together may form the same groups as R₁ and are preferably phenyl or substituted phenyl. Where substituents are present they may be methyl, ethyl, propyl or butyl, including t-butyl substituted, or may be methoxy, ethoxy, propoxy or butoxy including t-butoxy substituted. Particular compounds include

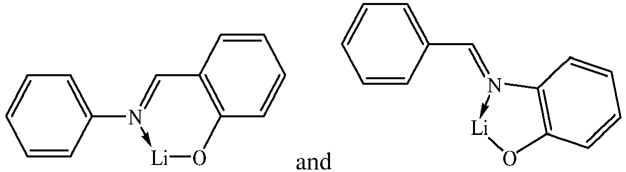

In many embodiments, aluminium is used as the cathode either on its own or alloyed with elements such as magnesium or silver, although in some embodiments other cathode materials e.g. calcium may be employed.. In an embodiment the cathode may comprise a first layer of alloy e.g. Li-Ag, Mg-Ag or Al-Mg closer to the electron injection or electron transport layer and a second layer of pure aluminium further from the electron injection or electron transport layer. Cells in which graphene serves as cathode are also within the invention.

The above compounds may be incorporated into a device which forms part of a flat panel display, a lighting panel, an organic photovoltaic device, an electrostatic latent image device, an organic thin film transistor, a dye sensitised solar cell device, a printed device, or a quantum dot based electroluminescent device. Such an OLED device may be used as a light source to print conductive, resistive, dielectric, piezoelectric or pyroelectric films or lines or grids.

It is believed that one or both of the compounds described herein can be incorporated into or used as a hole transport layer in a quantum dot electroluminescent device or QLED. More specifically, a representative QLED is described in US Patent Application No. 2009/009057 (Lee et al.,) which describes a QLED comprising: a substrate, a hole injection electrode, a hole transport layer, a quantum dot luminescent layer, an electron transport layer, and an electron injection electrode. Materials suggested for the hole transport layer include poly(3,4-ethylenedioxythiophene) (PEDOT)/polystyrene parasulfonate (PSS), poly-N-vinylcarbazole, polyphenylenevinylene, polyparaphenylene, polymethacrylate, poly(9,9-octylfluorene), poly(spiro-fluorene), N,N'-diphenyl-N,N'-bis 3-methylphenyl-1,1'-biphenyl-4,4'-diamine (TPD), N,N'-di(naphthalene-1-yl)-N-N'-diphenyl-benzidine, tris(3-methylphenylphenylamino)-triphenylamine (m-MTDATA), poly-9,9'-dioctylfluorene-co-N-(4-butylphenyl)diphenylamine (TFB), copper phthalocyanine, polyvinylcarbazole (PVK), and derivatives thereof and starburst materials. The quantum dot luminescent layer may include a material selected from the group consisting of a II-VI group compound semiconductor nanocrystal, a III-V group compound semiconductor nanocrystal, a IV-VI group compound semiconductor nanocrystal, a IV group compound semiconductor nanocrystal, and a mixture thereof; metal oxides, including ZnO, SiO₂, SnO₂, WO₃, ZrO₂, HfO₂, Ta₂O₅, BaTiO₃, BaZrO₃, Al₂O₃, Y₂O₃ and ZrSiO₄ and mixtures thereof. The present compounds or a mixture of them and optionally a p-dopant which is an acceptor-type organic molecule may be incorporated into the hole transport layer as alternative to, in addition to or in admixture with the compounds mentioned above, see also US Patent 7569248 (Jang et al., Samsung), US Patent No. 8552416 (Kin et al., L G Display Co.) and Kathirgamanathan, Electroluminescent Organic and Quantum Dot LEDs: The State of the Art, J. Display Technologies, 11, No. 5, 480-493. Such QLED devices may be incorporated into display panels or used for lighting.

How the invention may be put into effect will now be described with reference to the accompanying examples

### EXAMPLE 1 (Reference)

### Synthesis of 2,8-Dibromodibenzothiophene

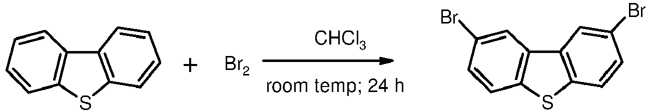

To a slurry of dibenzothiophene (15.0 g; 0.0814 mol) in chloroform (80 ml) was slowly added bromine (10.5 ml; 32.6 g; 0.18 mol) in chloroform (20 ml). The red-brown reaction mixture was stirred at room temperature for 24 h and then solvent removed by distillation at atmospheric pressure. The residue was washed three times with methanol until the filtrate became almost colourless. The off-white solid was dried under vacuum at 75 °C. Yield 20.5 g (74 %). The product was sublimed to give an analytically pure colourless solid, 18.2 g (89 %). M.p 223°C (DSC, onset) and 227°C (DSC, peak) [Lit., 226 °C, TCI]

### 2,8-Bis(1-thianthrenyl)dibenzothiophene [HTS-8]

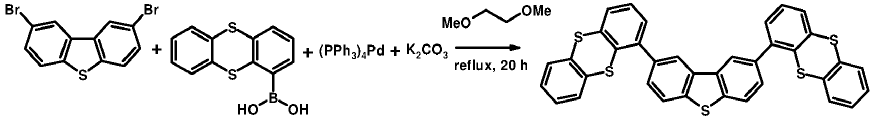

To a solution of 2,8-Dibromodibenzothiophene (1.0 g; 2.9 mmol) in ethyleneglycol dimethyl ether(50 ml) at 60°C was added 1-thianthreneboronic acid (1.6 g; 6.1 mmol) and tetrkis(triphenylphosphine)palladium (0.34 g; 0.29 mmol) followed by a solution of potassium carbonate (3.2 g; 23 mmol) in water (20 ml).The reaction mixture slowly darkened and became greenish in colour. The mixture was magnetically stirred and refluxed under nitrogen for 20 hours, after which solvent was removed from the cooled reaction mixture under reduced pressure. The residue was dissolved in dichloromethane (150 ml), extracted with water and then brine, after which the organic phase was dried over anhydrous magnesium sulphate. Removal of the solvent gave a greenish residue which was purified by column chromatography over silica gel using dichloromethane-petroleum ether (40-60 °C) (4:1) as eluent. A crude product was obtained by trituration with diethyl ether-petoleum ether (40-60 °C) to give an off-white solid, (1.3 g; 73 %) which was further purified by sublimation at 300°C (1.6 x 10⁻⁶ barr) to give a glassy solid, 0.48g which did not show any melting peak on the DSC. Its glass transition temperature Tg was 133 °C.
Found: C, 70.10; H, 3.41; S, 26.53.
C₃₆H₂₀S₅ requires C, 70.55; H, 3.29; S, 26.16 %.
UV: λmax/nm (ε) (CH₂Cl₂); 294(sh)(30,989), 263(97,473) and 230(35,934);
UV: λmax/nm (thin film); 303 and 268; Band gap: 3.85 eV
FL: λmax/nm(ε) (CH₂Cl₂); 441 (excitation wavelength: 350 nm),
   λmax/nm(ε) (powder) 439 (excitation wavelength: 350 nm);
   λmax/nm(ε) (thin film), 434, (excitation wavelength: 350 nm).
CV: electrolyte (100mM-Tetrabutylammonium tetrafluoroborate), analyte (1 mM),Solvent-dichloromethane: HOMO:-6.06 eV and LUMO:-2.2 eV Calculated from optical absorption edge of the thin film;
TGA/°C (% weight loss): 486 (5) and 510(10).

### Example 2

### 2-(4'-diphenylamino)phenyl-8-(1'-Thianthrenyl)-dibenzothiophene (HTS-11)

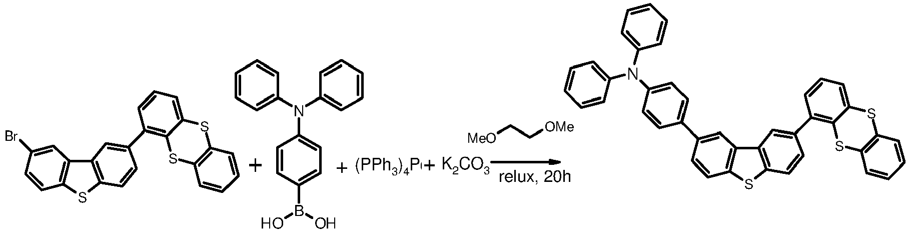

A mixture of 2-bromo-8-(1-thianthrenyl) dibenzothiophene (4.0 g, 8.38 mmole) and tetrakistriphenylphosphinepalladium (0.485 g; 0.420 mmol) was heated in 1,2-dimethoxyethane (50 ml) at about 50°C for 10 minutes. Then, 4-(diphenylamino)phenylboronic acid (2.2 g; 7.54 mmol) in 1,2-dimethoxyethane (10 ml) was added followed by potassium carbonate (5.8 g; 41.97 mmol) in water (10 ml). The resulting mixture was refluxed under nitrogen for 20 hours. The solvent removed from the cooled reaction mixture and the residue dissolved in dichloromethane and washed with water. The organic phase again washed with brine and dried over anhydrous magnesium sulphate and solvent removed to give the crude product. This was subjected to column chromatography over silica gel using CH₂Cl₂-Petroleum ether (40-60 °C). The eluent containing the product was evaporated using rotary evaporator and the residue was magnetically stirred with methanol overnight to give a fluorescent white solid, 3.3 g (61 %). The product was further purified by sublimation at 355°C and 2.0 x 10⁻⁶ Torr to give colourless glassy solid, 1.3 g, sublimation yield of around 40 %. The product did not show any melting peak in the DSC, but showed a Tg at 124°C
Found: C 79.37, H 3.67, N 2.45, S 16.04 %, also obtained C 77.52, H 4.13, N 1.89 and S 16.04 %.
C₄₂H₂₇NS₃, requires C 78.59, H 4.24, N 2.18 and S 14.99 %.
UV: λₘₐₓ (CH₂Cl₂)/nm (ε/dm³mol⁻¹ cm⁻¹) 338 (25,536) 291 sh(30,643) and 261(72,321).
λₘₐₓ (Thin film)/nm: 336 sh, 291(sh) and 261 nm.
Optical band gap: 3.26 eV.
FL: λₘₐₓ/nm(CH₂Cl₂)em: 413; ex/nm: 300; λₘₐₓ /nm(Powder)em: 419, ex/nm: 300; Xₘₐₓ/nm(Thin film) em: 414, ex/nm: 300.
TGA/ °C (% weight loss): 461 (1%) and 509 (5)

### Example 3 (Reference)

### 4-(1-thianthrenyl)-bis(triphenyl amine) [HTS-13]

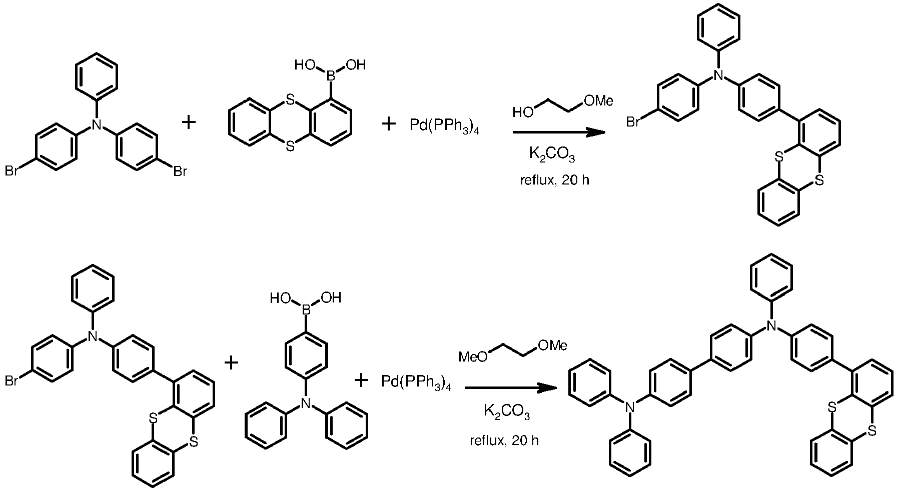

(a) To a slurry of 4,4'-dibromotriphenylamine (5.3 g; 0.013 mole) in 2-methoxyethanol (50 ml) was added tetrakis(triphenylphosphine) palladium (0.76 g; 0.00066 mole) followed by 1-thianthrenylboronic acid (3.42g; 0.013 mole) in 2-methoxy ethanol(30 ml). Then sodium tert-butoxide (2.4 g; 0.025 mole) was added followed by 2-methoxy ethanol (20 ml). The reaction mixture was refluxed under nitrogen for 20 hrs. The solution at the beginning became homogeneous then cloudy finally dark green in colour. The solvent removed under reduced pressure using the rotary evaporator and the residue extracted with dichloromethane. The dichloromethane solution was washed with brine and water then dried over anhydrous magnesium sulphate. The solvent after filtration of magnesium sulphate was evaporated in a rotary evaporator to give dark green solid. The solid was purified by column chromatography using petroleum ether- dichloromethane (3:2) to give a colourless solid, 3.4 g (48 %).
(b) 4-Bromo-4'-(1-thianthrenyl)triphenyl amine (3.3 g; 0.0061 mole) was taken-up in dimethoxy ethane (40 ml) and warmed at about 50°C for 10 min, then (PPh3)4Pd (0.75 g; 0.00065 mole) was added followed by toluene (20 ml). A clear solution was obtained but within 10 min it became dark in colour. 4-(diphenylamino)phenylboronic acid (1.60 g; 0.0055 mole) in toluene (20 ml) was added followed by potassium carbonate 92.6 g; 0.018 mole) in water (10 ml). The reaction mixture magnetically stirred and refluxed for 20 hrs. The solvent removed from the reaction mixture using rotary evaporator and the residue was dissolved in dichloromethane, washed with water twice and dried over anhydrous magnesium sulphate. The solvent filtered from magnesium sulphate and removed using rotary evaporator to give the crude product. The product was subjected to flash chromatography using CH₂Cl₂-Petroleum ether (40-60°C) [1:1] to give the product as viscous liquid. This was cooled and magnetically stirred with methanol and small amounts of petroleum ether (40-60°C) to give an amorphous white solid. The solid was filtered off and dried under vacuum at 80 °C to give 3,1 g of product (72 %). The product was further purified by sublimation at 280°C (4.8 x 10-7 Torr) to give a glassy solid 1.3 g (42 %). The compound did not show any melting peak in DSC, but showed a glass transition temperature (Tg) at 107 °C.
   Found: C 81.20, H 4.80, N 3.93, S 10.16 %.
   C48H34N2S2 requires, C 82.02, H 4.88, N 3.98 and S 9.12 %.
   UV: λₘₐₓ (Toluene)/nm (ε/dm³ mol⁻¹ cm⁻¹) 324.50 (36,506).
   λₘₐₓ (Thin film)/nm: 324.50 and 265.50 nm.
   Optical band gap: 3.1 eV.
   FL: λₘₐₓ/nm(toluene)em: 394 and 410(sh); ex/nm: 320;
   λₘₐₓ/nm(Powder)em: 427, ex/nm: 320; λₘₐₓ /nm(Thin film) em: 428 and 410 (sh), ex/nm: 320.
   TGA/ °C (% weight loss): 382 (5) and T_{d} at 498 °C.

### Example 4

### Efficiency of OLED devices

Oleds were fabricated using as hole transport layers α-NPB, HTS-8 and HTS-11. The layers were as follows (HIL means hole injection layer, HTL menas hole transport layer, EML means emissive layer and ETL means electron transport layer):

| | |
|---|---|
| Anode: | ITO on glass |
| HIL: | PEDOT:PSS ∼40 nm, spin coated |
| HTL | NPB (50 nm), HTS-8 (10-22 nm) or HTS-11 (10-30 nm) |
| EML: | TCTA:TPBi (3:7) : Ir(ppy)2acac, doping 10% |
| ETL: | TPBi (30 nm) |
| Cathode: | LiF (0.5nm) / Al (100nm) |

In the above listing PEDOT means poly(3,4-ethylenedioxythiophene) and PSS means poly(4-styrenesulfonate), TCTA means tris(4-carbazoyl-9-ylphenylamine) and TPBi means 1,3,5-tris(N-phenylbenzimidizol-2-yl)benzene. The PEDOT:PSS is spin coated and the HTS-8 is also spin coated in chlorobenzene. Device performance for various cells is shown below:

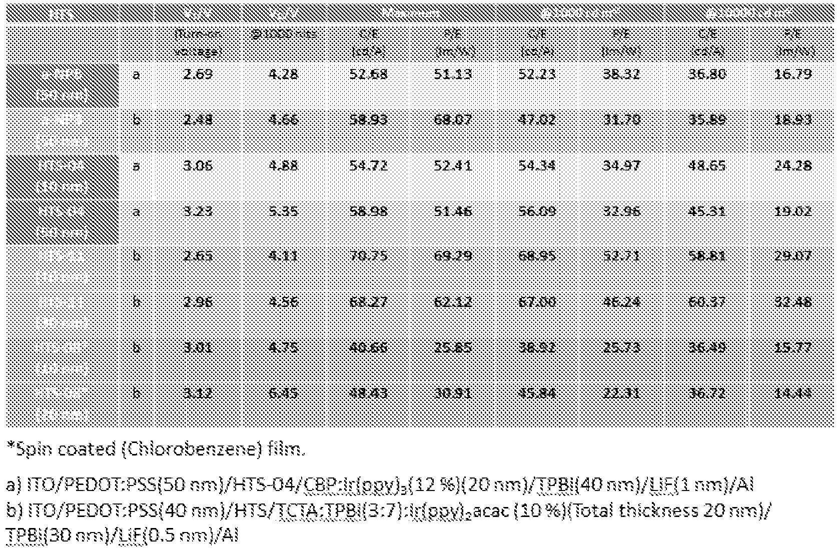

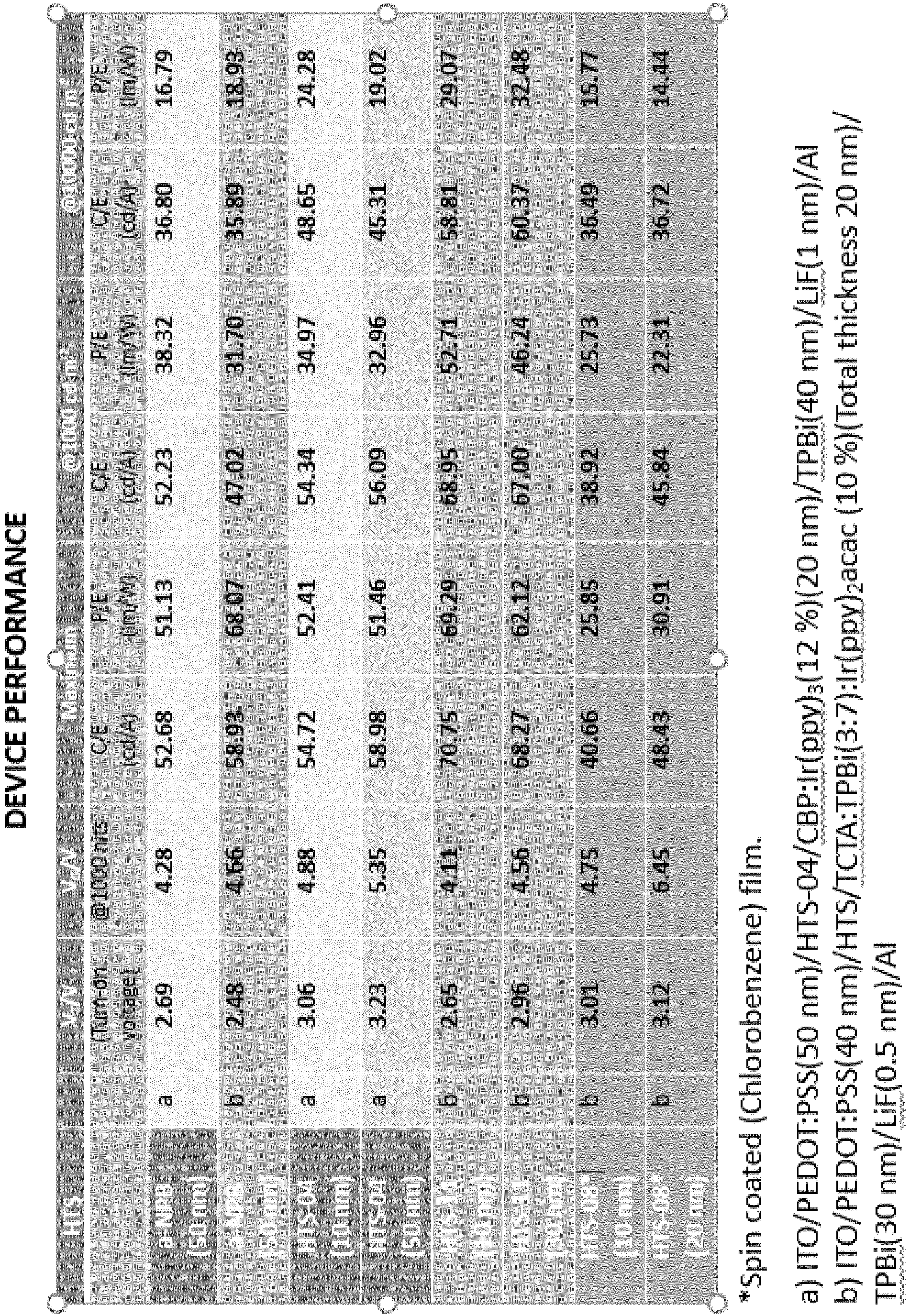

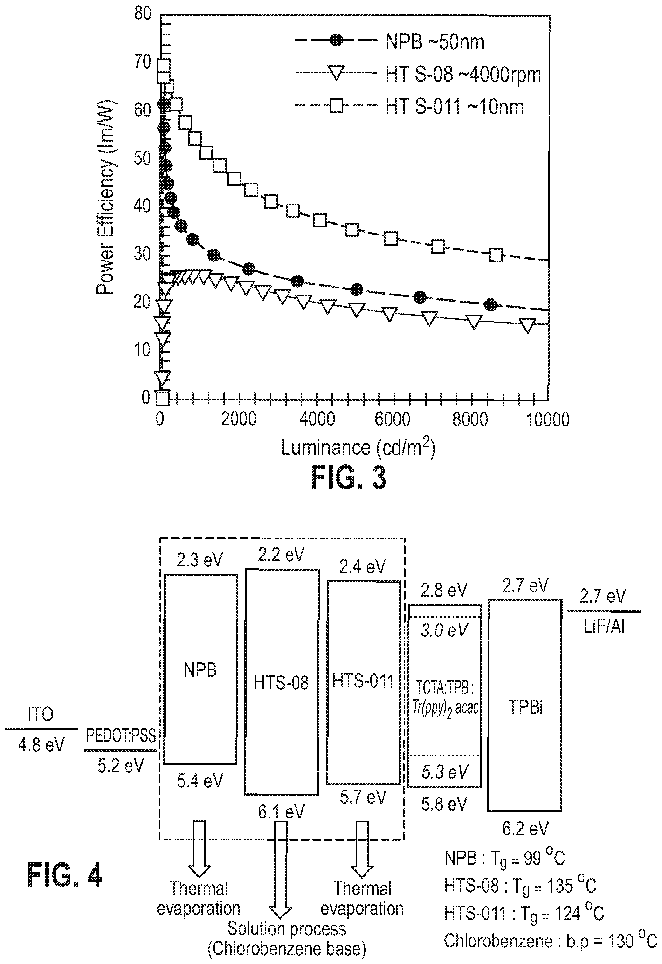

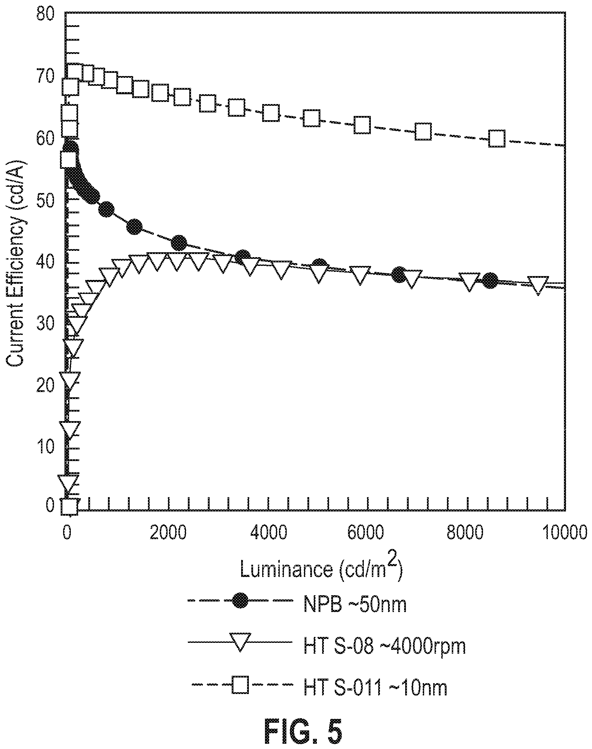

The cells provide green emission. Efficiency in a representative cell is shown in Fig 3 and is in the order HTS-11 > HTS-8 > α-NPB and an energy band diagram for the cell appears as Fig. 4. A plot of current efficiency against luminance appears as Fig. 5.

## Claims

1. 2-(4'-Diphenylamino)phenyl-8-(1'-thianthrenyl)-dibenzothiophene.

2. A hole transport material comprising the compound of claim 1 and a p-dopant which is an acceptor-type organic molecule.

3. The material of claim 2, wherein the dopant is present in an amount such that when the material is deposited to form a layer the dopant contributes 10-40% to layer thickness.

4. The material of claim 3, wherein the dopant is present in an amount such that when the material is deposited to form a layer the dopant contributes 33% to layer thickness.

5. The material of any of claims 2-4, wherein the dopant comprises any of:
(a) tetracyanoquinodimethane or tetrafluorotetracyano-quinodimethane.
(b) a compound of any of the general formulae (i) - (v) below: 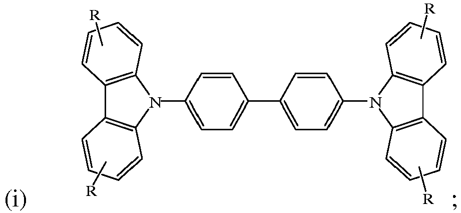 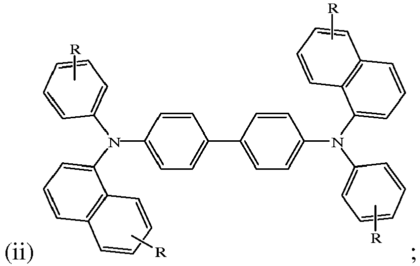 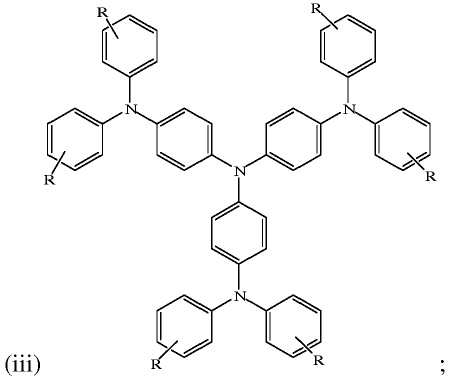 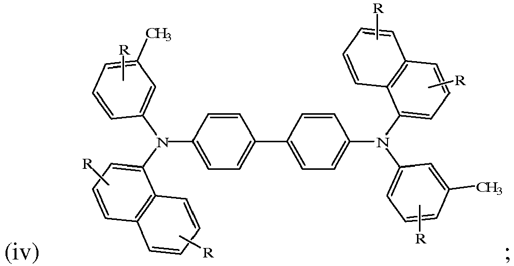 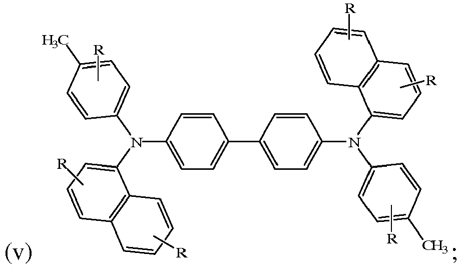
wherein the groups R in any of the formulae in (i) to (v) can be the same or different and are selected from hydrogen; substituted and unsubstituted aliphatic groups; substituted and unsubstituted aromatic, heterocyclic and polycyclic ring structures; halogens; and thiophenyl groups; and wherein
in formula (i) the methyl groups may be replaced by C₁-C₄ alkyl or monocyclic or polyclic aryl or heteroraryl which may be further substituted e.g. with alkyl, aryl or arylamino, or of the formula viii or ix 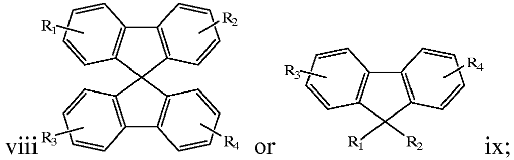
wherein the groups R₁-R₄ when appearing in either of the above formulae can be the same or different and are selected from hydrogen; substituted and unsubstituted aliphatic groups; substituted and unsubstituted aromatic, heterocyclic and polycyclic ring structures; halogens; and thiophenyl groups.
(c) a teritiary amine which is α-NPB or β-NBP.

6. An optical light-emitting diode having first and second electrodes and between said electrodes a layer comprising a compound as claimed in claim 1 or a material as claimed in any of claims 2-5.

7. The device of claim 6, wherein said layer is a hole transport layer.

8. The device of claim 6 or 7, having an emissive layer comprising a fluorescent emitter, a phosphorescent emitter, an ion fluorescent (rare earth based) emitter, quantum dots or a thermally activated fluorescent (TADF) material.

9. The device of any of claims 6-8, having a hole injection layer comprising CuPC, ZnTpTP, 2-TNATA or

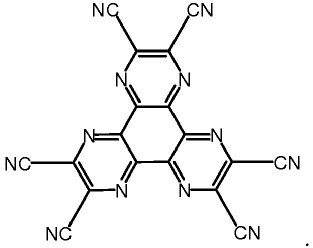

10. The device of any of claims 6-9, which forms part of a flat panel display or a lighting panel.

11. Any of the following including a compound as claimed in claim 1 or a material as claimed in any of claims 2-5:
an organic photovoltaic device;
an imaging member for forming an electrostatic latent image;
an organic thin film transistor;
a dye sensitised solar cell device;
a printed device;
a quantum dot based electroluminescent device;
an OLED device used as a light source to print conductive, resistive, dielectric, piezoelectric or pyroelectric films or lines or grids;
an OLED lighting panel.

## Patentansprüche

1. 2-(4'-Diphenylamino)pheny1-8-(1'-thianthrenyl)-dibenzothiophen.

2. Lochleitungsmaterial, umfassend die Verbindung nach Anspruch 1 und einen p-Dotierstoff, der ein organisches Molekül des Akzeptortyps ist.

3. Material nach Anspruch 2, wobei der Dotierstoff mit einer solchen Menge vorliegt, dass, wenn das Material abgeschieden wird, um eine Schicht zu bilden, der Dotierstoff 10-40 % der Schichtdicke ausmacht.

4. Material nach Anspruch 3, wobei der Dotierstoff mit einer solchen Menge vorliegt, dass, wenn das Material abgeschieden wird, um eine Schicht zu bilden, der Dotierstoff 33 % der Schichtdicke ausmacht.

5. Material nach einem der Ansprüche 2-4, wobei der Dotierstoff irgendeines aus dem Folgenden umfasst:
(a) ein Tetracyanoquinodimethan oder Tetrafluortetracyanoquinodimethan.
(b) eine Verbindung mit irgendeiner der nachstehenden allgemeinen Formeln (i)-(v): 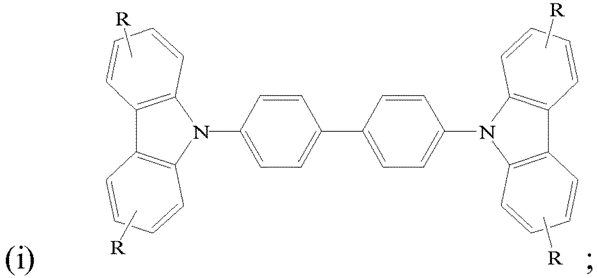 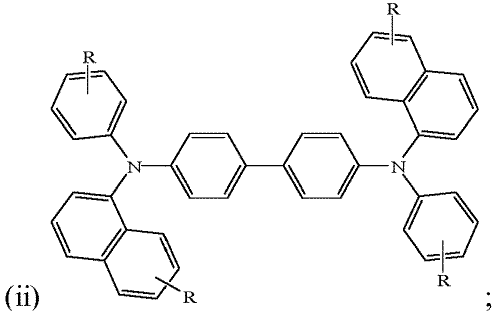 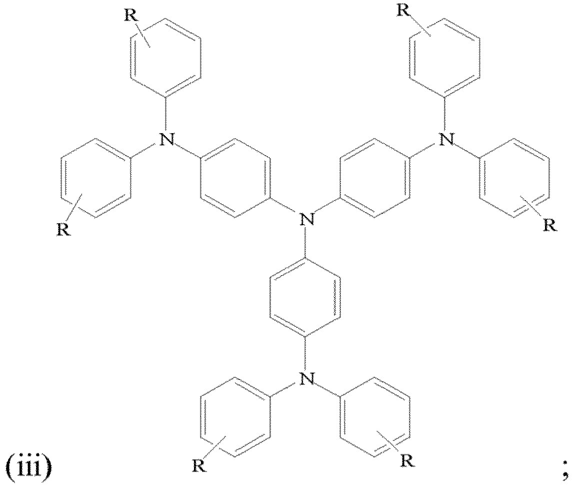 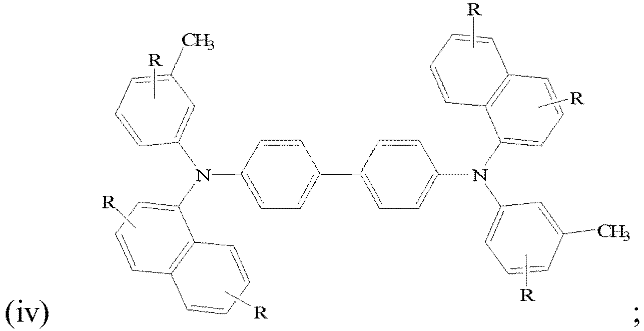 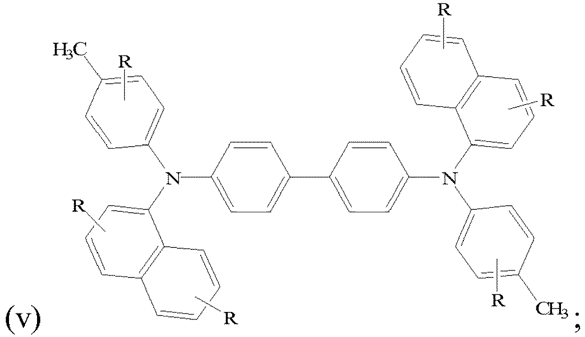
wobei die Gruppen R in irgendeiner der Formen (i) bis (v) gleich oder verschieden sein können und aus Wasserstoff; substituierten oder unsubstituierten, aliphatischen Gruppen; substituierten und unsubstituierten, aromatischen, heterocyclischen und polycyclischen Ringstrukturen; Halogenen; und Thiophenylgruppen ausgewählt sind; und wobei
in der Formel (i) die Methylgruppen durch C₁-C₄ Alkyl oder monocyclisches oder polycyclisches Aryl oder Heteroalkyl ersetzt sein können, die ferner mit z. B. Alkyl, Aryl oder Arylamino substituiert sein können, oder der Formel viii oder ix 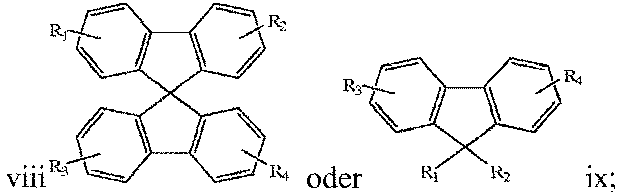
wobei die Gruppen R₁-R₄, wenn sie in irgendeiner der oben genannten Formeln auftreten, gleich oder verschieden sein können und aus Wasserstoff; substituierten und unsubstituierten, aliphatischen Gruppen; substituierten und unsubstituierten, aromatischen, heterocyclische und polycyclischen Ringstrukturen; Halogenen; und Thiophenylgruppen ausgewählt sind.

6. Optische, lichtemittierende Diode, die eine erste und eine zweite Elektrode und zwischen den Elektroden eine Schicht aufweist, die eine Verbindung nach Anspruch 1 oder ein Material nach einem der Ansprüche 2-5 umfasst.

7. Vorrichtung nach Anspruch 6, wobei die Schicht eine Lochleitungsschicht ist.

8. Vorrichtung nach Anspruch 6 oder 7, die eine Emitterschicht aufweist, die einen phosphoreszierenden Emitter, einen ionenfluoreszierenden Emitter (auf Seltenerdbasis), Quantumpunkte oder ein thermisch aktiviertes, fluoreszierendes (TADF) Material umfasst.

9. Vorrichtung nach einem der Ansprüche 6-8, die eine Lochinjektionsschicht aufweist, umfassend CuPC, ZnTpTP, 2-TNATA oder

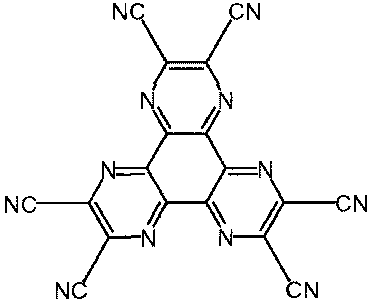

10. Vorrichtung nach einem der Ansprüche 6-9, die einen Teil eines Flachbildschirms oder eines Leuchtpanel bildet.

11. Eines aus dem Nachstehenden, umfassend eine Verbindung nach Anspruch 1 oder ein Material nach einem der Ansprüche 2-5:
eine organische Fotovoltaikvorrichtung;
ein Abbildungselement zum Bilden eines elektrostatischen, latenten Bildes;
einen organischen Dünnfilmtransistor;
eine farbstoffsensibilisierte Solarzellenvorrichtung;
eine gedruckte Vorrichtung;
einer auf einem Quantenpunkt basierende Elektrolumineszenzvorrichtung;
eine OLED-Vorrichtung, die als eine Lichtquelle zum Drucken von leitfähigen, resistiven, dielektrischen, piezoelektrischen oder pyroelektrischen Filmen oder Linien oder Gitter verwendet wird;
ein OLED-Leuchtpanel.

## Revendications

1. 2-(4'-diphénylamino)phényl-8-(1'-thianthrényl)-dibenzothiophène.

2. Matériau de transport de trou comprenant le composé de la revendication 1 et un dopant p qui est une molécule organique de type accepteur.

3. Matériau selon la revendication 2, dans lequel le dopant est présent dans une quantité telle que le matériau est déposé pour former une couche pour laquelle le dopant contribue à l'épaisseur de couche à hauteur de 10 à 40 %.

4. Matériau selon la revendication 3, dans lequel le dopant est présent dans une quantité telle que le matériau est déposé pour former une couche pour laquelle le dopant contribue à l'épaisseur de couche à hauteur de 33 %.

5. Matériau selon l'une quelconque des revendications 2 à 4, dans lequel le dopant comprend l'un quelconque parmi :
(a) le tétracyanoquinodiméthane ou le tétrafluorotétracyano-quinodiméthane,
(b) un composé de l'une quelconque des formules générales (i) à (v) ci-dessous : 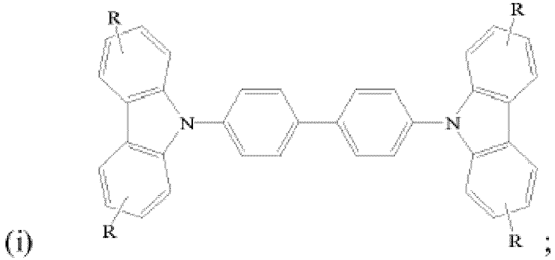 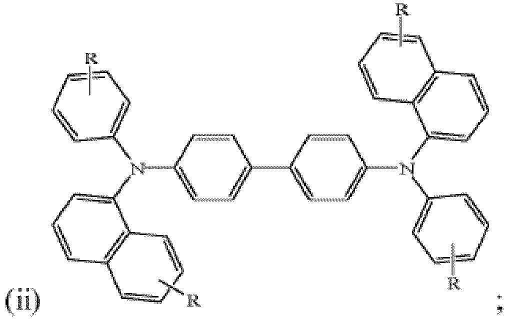 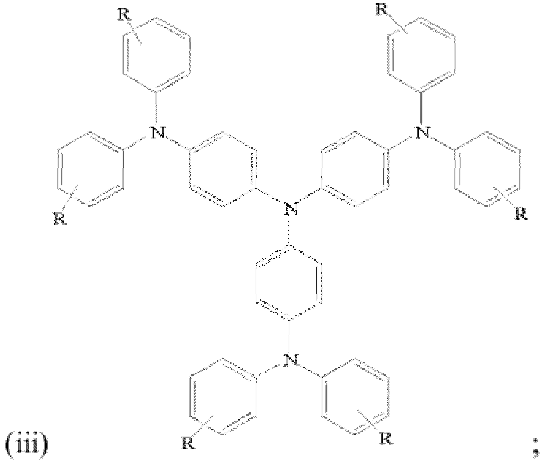 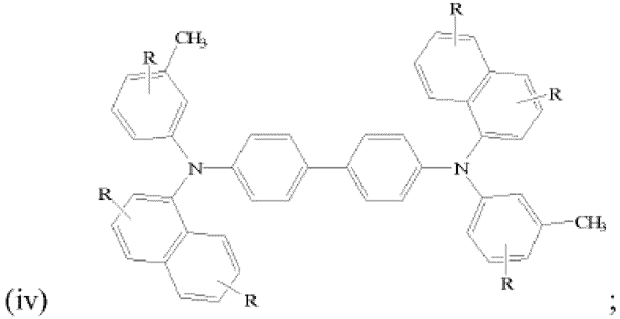 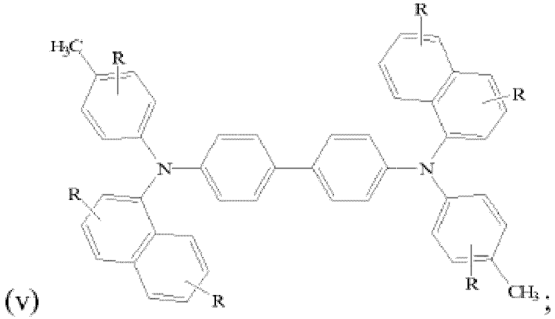
dans lequel les groupes R dans l'une quelconque des formules (i) à (v) peuvent être identiques ou différents et sont choisis parmi hydrogène ; des groupes aliphatiques substitués et non substitués ; des structures de cycles aromatiques, hétérocycliques et polycycliques substitués et non substitués ; des halogènes ; et des groupes thiophényle ; et dans lequel
dans la formule (i) les groupes méthyle peuvent être remplacés par un alkyle en C₁ à C₄ ou aryle ou hétéroaryle monocyclique ou polycyclique qui peut être encore substitué par, par exemple, alkyle, aryle ou arylamino, ou de formule viii ou ix 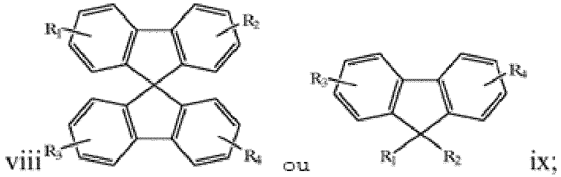
dans lequel les groupes R₁ à R₄ lorsqu'ils apparaissent dans l'une ou l'autre des formules ci-dessus peuvent être identiques ou différents et sont choisis parmi hydrogène ; groupes aliphatiques substitués et non substitués ; structures de cycles aromatiques, hétérocycliques et polycycliques substitués et non substitués ; halogènes ; et groupes thiophényle.

6. Diode électroluminescente optique ayant des première et seconde électrodes et entre lesdites électrodes, une couche comprenant un composé tel que revendiqué dans la revendication 1 ou un matériau tel que revendiqué dans l'une quelconque des revendications 2 à 5.

7. Dispositif selon la revendication 6, dans lequel ladite couche est une couche de transport de trou.

8. Dispositif selon la revendication 6 ou 7, ayant une couche émissive comprenant un émetteur fluorescent, un émetteur phosphorescent, un émetteur fluorescent ionique (à base de terres rares), des points quantiques ou un matériau fluorescent activé thermiquement (TADF).

9. Dispositif selon l'une quelconque des revendications 6 à 8, comportant une couche d'injection de trou comprenant CuPC, ZnTpTP, 2-TNATA ou

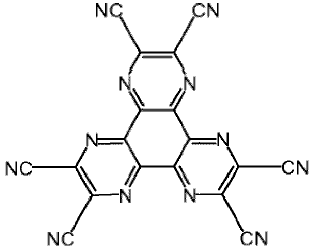

10. Dispositif selon l'une quelconque des revendications 6 à 9, qui fait partie d'un écran plat ou d'un tableau d'éclairage.

11. Elément quelconque parmi les suivants comprenant
un composé tel que revendiqué dans la revendication 1 ou un matériau tel que revendiqué dans l'une quelconque des revendications 2 à 5 :
un dispositif photovoltaïque organique ;
un organe d'imagerie destiné à former une image latente électrostatique ;
un transistor à couches minces organique ;
un dispositif de cellule solaire à colorant ;
un dispositif imprimé ;
un dispositif électroluminescent à base de points quantiques ;
un dispositif DELO utilisé comme source de lumière pour imprimer des films, ou lignes, ou grilles conducteurs (conductrices), résistifs (résistives), diélectriques, piézoélectriques ou pyroélectriques ;
un tableau d'éclairage à DELO.
